# EUROPEAN PATENT SPECIFICATION

(11) **EP 1 999 405 B1**
(45) Date of publication and mention of the grant of the patent: **22.07.2009**
(21) Application number: 07736734.0
(22) Date of filing: 28.03.2007
(51) Int. Cl.: F21S 8/00

(54) **LIGHTING DEVICE PROVIDED WITH AUTOMATIC AIMING SYSTEM**
BELEUCHTUNGSVORRICHTUNG MIT EINEM AUTOMATISCHEN ZIELSYSTEM
DISPOSITIF D'ÉCLAIRAGE DOTE D'UN SYSTÈME DE POINTAGE AUTOMATIQUE

(30) Priority: 29.03.2006 IT NA20060039
(43) Date of publication of application: 10.12.2008
(73) Proprietor: ASImmobiliare s.r.l., 00145 Roma (IT)
(72) Inventor: SPADARO, Antonio, I-00144 Roma (IT)
(86) International application number: PCT/IT2007/000230
(87) International publication number: WO 2007/110895

(56) References cited:
- DE-U1- 20 316 756
- US-A- 5 038 261
- US-A- 5 068 767

## Description

### TECHNICAL FIELD

The present invention relates to a lighting device provided with automatic aiming system. In particular, the lighting device concerns a scialytic lamp, i.e. a lamp that is used in surgery for the illumination of an operating field.

### BACKGROUND ART

In the field of the surgical procedures the most important requirements of a scialytic lamp are a perfect brightness control in the operating field, no shadow areas and a constant level of brightness. These requirements, that have never been achieved completely until now, are critical even more in the highly specialised surgery, such as the neurosurgery, and in all the procedures performed with microsurgical technique, where the dimensions of the operating field and of surgical instruments are reduced. For this reason in microsurgery it is required that an absolutely constant brightness follows the operating surgeon in all his/her manoeuvres without either hindrance of movements thereof or risk of pollution in the operating field.

US Patent No. 4,884,008 to Bossler et al. discloses an auto-adjustment illumination for an operating room in accordance with a manual positioning of the light, wherein a feedback-loop servo- mechanism positions light sources or mirrors which are contained in a lamp-holder to illuminate a given field in a given plane. The distance of the lamp from the given field is determined by an ultrasonic distance sensor to provide an input to the feedback loop, for comparison of the positioning of the light source to illuminate the field. If the distance of the illuminated field increases, the angular position of the light sources is automatically adjusted. If the distance decreases, no adjustment is carried out unless an operator touches the lamp. This is transformed into a new signal of manual adjustment which releases the servo system to then readjust the position of the light sources.

US Patent No. 5,383,105 to Agut discloses a lamp for surgical illumination able to automatically adjust the concentration of the light rays on an operating field, that are provided by at least a reflector and at least a light source. The automatic adjustment is performed by a relative movement between reflector and lamp as a function of the outcome of comparison between the current algebraic value of the measurement of the luminous intensity of the light reflected by the operating field, with that of a previously performed measurement. The light intensity is measured with a device which is sensitive to the light and is accommodated in a lamp gripping handle. Touching of a sensor on the handle actuates a cycle of adjustment, in the course of which a plurality of measurements of the luminous intensity and displacements of the light source are performed jointly with the displacement of the light source, to arrive at the optimal optical concentration.

The above mentioned lighting devices are designed to adjust the concentration of the light rays depending on the distance between the lighting devices and the operating field, and the lighting devices are aimed manually.

Instead there are other lighting devices whose purpose is to perform an optimal illumination on an operating field. Among these, a device, that is disclosed in US Patent No. 5,038,261, has a cardanic mounting connected to an overhead beam by joints which rotate about horizontal and vertical axes. After setting of the optimal illumination zone into the operating plane, that zone's co-ordinates, and the associated co-ordinates of the lamp housing, are stored as values in a computer. Upon intentional or unintentional spatial displacement of the lamp housing, angle sensors in the joints, and a distance sensor in the lamp housing, furnish data to the computer, which calculates a compensating adjustment and carries it out by applying control signals to positioning motors in the cardanic mounting, until the axis of the light beam again place the optimal illumination on the operating field.

US Patent No. 5,038,261 discloses the use of an ultrasound sensor. However, US Patent No. 5,068,767 discloses an automatic focus position adjusting unit using an optical sensor.

As it will be clear in the following, the present invention can be considered close to US Patent No. 5,038,261 since, as in the latter, the invention has a fixed supporting element, at least a sensor, a computer that receives data from the sensor and uses it to control the operation of positioning motors for the aiming onto the operating field.

However, this invention is enhanced by technical features that have their base in the neuroanatomical and functional organisation of the structures designed to spatial conjugate ocular vision.

Really, the orientation of a living creature eye in the vision mechanism is adjusted by functional systems which are provided by such a synergism that the fixation of "target" objects is assured, also when the latter are moving in the space.

The complex system of the ocular pursuit movement has a primary function of maintaining constantly the fovea, i.e. the sensitive retinal part, collimated onto a target, upon the localisation thereof.

The eye, as a receptor, is subjected to continuous and automatic small movements, which are both slow and fast, also during the fixation of static targets. As a result of these continuous ocular adjustments a continuous, constant, collimated relationship between foveal point and target is maintained. The retinal cell is the first sensorial receptor, the optic nerve is a conduction beam of afferent signal, the superior quadrigeminal colliculi act as a kind of sorting centres, the cerebral cortex is the "smart" integration centre and the oculomotor nucleuses function as efferent centres for the ocular motricity.

The Applicant of this invention has gained from the natural operation of the eye a mechanism suitable to collimate a light beam to an aim or target positioned in a defined receiving field.

An object of the invention is to overcome the drawbacks of the prior art that are, on one hand, the difficulty of achieving the above mentioned requirements for a scialytic lamp, and, on the other hand, the complexity of the existing lighting devices for operating-rooms.

### DISCLOSURE OF INVENTION

According to the present invention there is provided a lighting device provided with automatic aiming system, comprising a fixed supporting element and a movable lamp-holder holding light sources that are fixed thereto, driving means adapted to move the movable lamp-holder with respect to the fixed supporting element, a sensor and a micro-controller which receives data from the sensor and uses it to control the operation of said driving means in order to modify the position of the movable lamp-holder and then the orientation of light beams thereof in the space, **characterised in that** said sensor is an optical sensor adapted to detect a reflectance factor of the light from an illuminated field for each position of the lamp-holder, the lighting device also including an optical encoder able to provide the position of the movable lamp-holder in every moment, the micro-controller receiving from the optical sensor data relevant to such a reflectance factor, and from the optical encoder data relevant to the position of the movable lamp-holder, to control the operation of driving means for the constant aiming of a zone of the illuminated field which has a maximum reflectance factor.

### BRIEF DESCRIPTION OF DRAWINGS

The present invention will be described with reference to a preferred embodiment thereof, in connection with the enclosed drawing, in which:
Figure 1 shows an underneath plan view of a lighting device provided with automatic aiming system according to an embodiment of the present invention;
Figure 2 shows a side view of the lighting device in Figure 1, without lateral cover;
Figure 3 shows a side view of the lighting device in Figure 1, yet without lateral cover, but 90 degrees rotated and fixed to a ceiling;
Figure 4 is a block diagram of the system that allows an automatic aiming of the lighting device; and
Figure 5 is a flowchart depicting the logic of the system with reference to the block diagram in Figure 4.

### BEST MODE FOR CARRYING OUT THE INVENTION

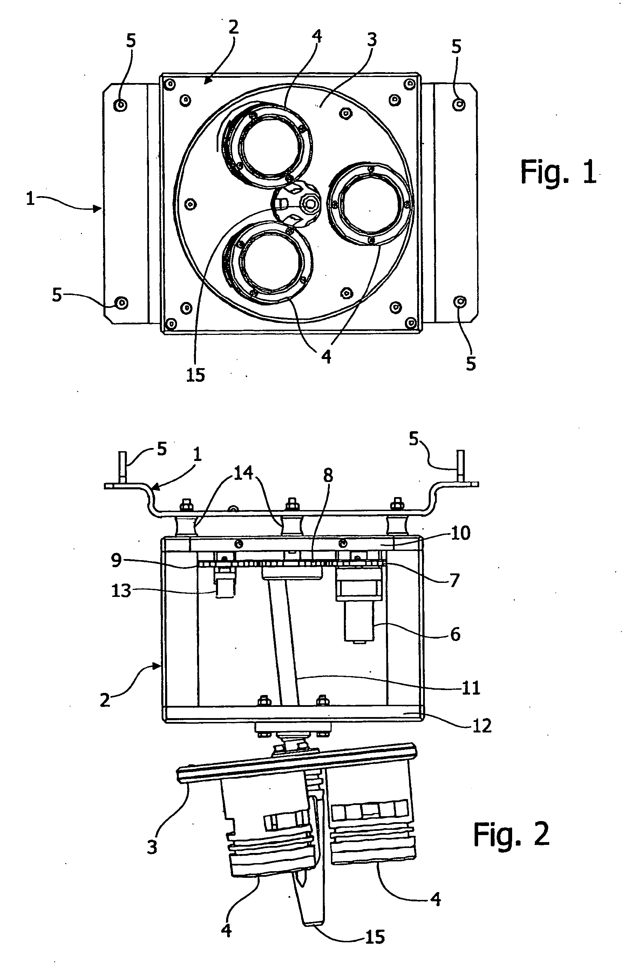

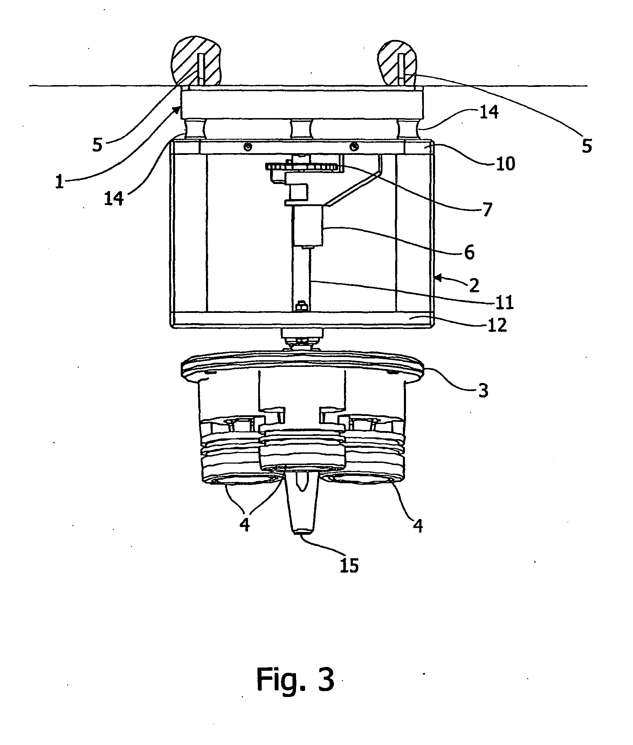

First, reference is made to Figures 1 to 3, which are orthogonal views of an embodiment of the lighting device according to the invention, being an integral part of a scialytic lamp for operating-room. In Figures 1 to 3, a fixed support element is indicated as 1, a frame as 2, and a movable lamp-holder denoted by 3 is in the form of a disk on which three lamps with reflectors and indicated generally as 4 are mounted at the same angular distance. The fixed support element 1 is designed to be applied to a ceiling, as shown in Figure 3, by means of screws indicated as 5.

The frame 2 is designed to sustain an aiming mechanism for the movable lamp-holder 3. Driving means for moving the lamp-holder 3 with respect to the fixed support element 1 is part of the aiming mechanism.

With reference to Figure 2 particularly, the driving means comprises an electric motor 6 having a shaft to which a driving gear wheel 7, being meshed with a first driven gear wheel 8, is keyed. Suitably, the gear wheels 7 and 8 have the same number of teeth and the same pitch. A second driven gear wheel 9, having the same number of teeth and the same pitch of the first two gear wheels, is meshed with the first driven gear wheel 8 so that the second driven gear wheel 9 rotates at the same speed and in the same direction of the driving gear wheel 7. The above described driving means are mounted to an upper plate 10 of the frame 2. The frame 2 is shown as an open parallelepipedal construction, but obviously it is closed by suitable panels on its lateral surface.

Eccentrically pivoted to the first driven gear wheel 8 is an end of a rod 11, whose opposite end is pivoted to the lamp-holder disk 3 by means of a ball joint. The rod 11 is constrained to rotate, in a position between its ends, on a lower plate 12 of the frame 2.

An optical encoder 13, which is adapted to give the position of the movable lamp-holder, is mounted to the second driven gear wheel 9.

The frame 2 is mounted to the supporting element 1 through its upper frame plate 10 by interposed vibration-damping pads, which are generally indicated as 14 and serve to damp vibrations generated by the motor and permit more accuracy of the light beam while it is moving.

Preferably, centrally mounted to the lamp-holder disk is an optical sensor 15, whose function will be evident from the following of the description.

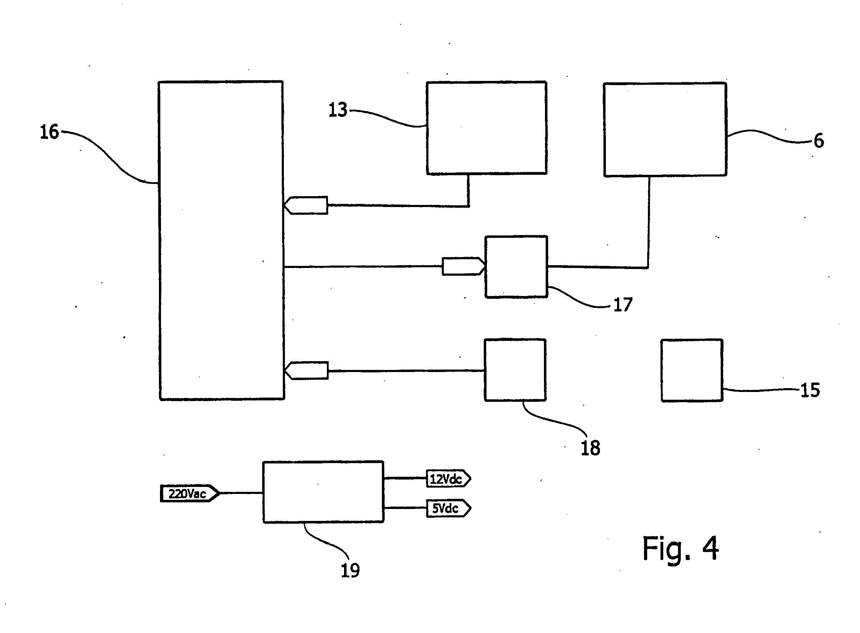

Referring to Figure 4, there is shown a block diagram of the aiming system, which the lighting device according to the present invention is provided with. Blocks are denoted by some of the reference numerals above cited, such as the motor 6, the optical encoder 13 and the optical sensor 15. Further, a micro-controller, which is indicated as 16, is connected to the motor 6 by a power amplifier 17 and to the optical sensor 15 by an amplifier 18. Furthermore, a power source 19 is provided.

Now, driving means operates as follows. The driving gear wheel 7, through the motion given by the motor 6 whose shaft supports keyed the driving gear wheel 7, begins to move transmitting the movement to the driven gear wheel 8, that in turn will give the rotary movement also to the rod 11, which has one end pivoted to the lamp-holder disk 3 and the other end pivoted to the driven gear wheel 8. The combination of the rotary movement of the rod 11, which is constrained near the one end thereof, generates a rotation describing a cone in the space. The rod 11, being connected to the lamp-holder disk 3, will transmit this kind of movement also to the latter. Further the first driven gear wheel 8 has a double purpose: besides generating a conic-rotary movement, it transmits its rotation also to the second driven gear wheel 9, which in turn is connected to the optical encoder 13. Preferably, the three gear wheels 7, 8, and 9 has the same number of teeth and the same pitch, then their rotation speed is the same, whereas the direction of rotation is the same for the driving gear wheel 7 and the second driven gear wheel 9 for the encoder 13. In this way there is a biunivoc correspondence between the shaft of the motor 6 and the encoder 13, as they move always at the same rotation time and at the same speed.

Preferably, three 50 W halogen lamps 4 are mounted to the lamp-holder disk 3 according to the invention. These lamps project a spot onto a work plane situated at a distance of about two meters, the spot having such an intensity that it assures a brightness prescribed for a micro-precision operation. The lamps are mounted to the lamp-holder disk 3 with a determined angle toward the central axis of the lamp-holder disk 3 so that beams of the three lamps converge to a same point from a distance of two meters. The angle chosen is about 2 degrees, more precisely 1 degree 51 min 36 sec (0,0264 radian).

The mechanical design of the lamp-holder disk forces the spot being projected by the lamps to move along an elliptic trajectory, permitting to aim the light beam onto any point inside the ellipse by rotating the axis of the lamp-holder disk.

In order to keep the aiming onto a target movable on a plane that is at a predetermined distance, the device must be interlocked to an automatic system able to locate the point in question and orient the lamps so that they illuminate it.

The interlocking system of the device is schematised in Figure 4.

It comprises the direct-current motor 6, having a power suitable to move the rotating lamp-holder disk 3, the amplifier/polarity inverter 17 to control the motor 6 and invert the running direction thereof, the optical encoder 13 for the angular position, the optical sensor 15 with the signal amplifier 18, and finally an electronic card based on the micro-controller 16, i.e. a microprocessor that is specialised to control systems and is provided, on one chip, with all necessary resources as well as a real processing unit.

The microprocessor 16 controls the position of the lamp-holder disk until it optimises the illumination of a concerned zone that must have a reflectance or reflection factor greater with respect to the rest of the scenery. Such a restraint is necessary since the system must distinguish the concerned zone from the rest of the scenery on the base of some parameter. Suitably, the value of the light reflection factor has been chosen as a distinguishing parameter. In practice, it is sufficient that the working field is clearer than the contour, that must have a darker colour. This parameter is usual in the operating rooms, where sheets are chosen of a dark colour, and also gloves of the operating surgeons are opaque.

The aiming system will search for the point with greater reflection factor on the working plane, and it will be a task of the user to make so that this condition is respected.

A brightness feedback is furnished by the accurate optical sensor 15, which is housed in the centre of the lamp-holder disk 3 and has a visual field that through a small-diameter eyepiece is reduced to a portion of the illuminated zone of about 15 cm in diameter, this portion being also centred with respect to the axis of the lamp-holder disk 3. An analog signal from the optical sensor 15 is amplified in situ to limit electric noises and delivered through a shielded wire to an analog-digital converter inside the micro-controller. Such a converter serves to transform the brightness data item into a number, which is stored in an internal memory of the micro-controller.

The axis of the lamp-holder disk, which is keyed with the optical encoder with a mechanical ratio 1:1, allows the micro-controller to know the position of the rod in every moment. In this way, the micro-controller can store in its internal memory brightness data bound to the angular position of the lamp-holder disk 3, position that is related univocally to an exact point of the working plane. This will permit the maximum brightness position to be found. In the system there is finally the suitable power source 19, which furnishes voltages required by electronics and lamps, from the main voltage.

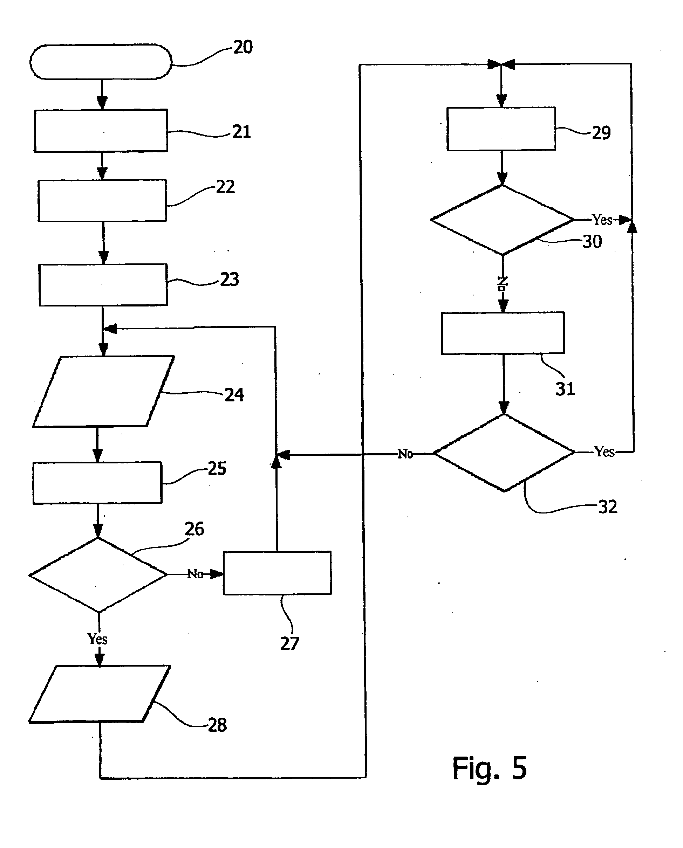

The logic of operation of the micro-controller, implemented in its internal program, is synthetized in the flowchart in Figure 5.

After initial set-up in a start step 20, reset of the internal variables 21, IN/OUT setting 22, and interrupt activation 23, the micro-controller 16 begins an acquisition procedure consisting of making a complete turn of the lamp-holder disk 3 (step 24) as well as acquiring brightness data of the framed scenery, which is divided into 256 sections. Brightness data item of every section is related to its angular position, by virtue of information provided by the optical encoder 13. When the turn is finished, the micro-controller searches, in a string of data stored inside, the maximum brightness value (step 25), together with its angular position. On the base of the assumed condition, such a maximum value coincides with the zone in which there is the target to be illuminated, whereby the micro-controller, by operating the motor, positions the lamp-holder disk in the direction of this maximum value. The positioning movement follows an elliptic trajectory and the direction of rotation is calculated for following the shortest path.

However, it can happen in a decision step 26 that the brightness measure performed during the turn does not contain the required information: this occurs for example when the lamps are switched off or the scenery does not present a sufficient contrast between clear and dark zones (the reflectance is almost uniform). In this case, the micro-controller detects the abnormal condition since, beside the maximum brightness value, it calculates also the minimum one, and if the difference does not exceed a pre-set value, the measure is deemed invalid and after 2 seconds it will be repeated (step 27).

In a case of correct acquisition, after the positioning on the target (step 28), the system gets into stand-by and performs periodically brightness measures in the point in order to verify the continue centring of the target in a two second waiting step 29, to go to a decision step 30 where the question is if the target is centred, to a step 31 for performing a local research and to a decision step 32.

Really, if the light level goes down a determined threshold, which is calculated as a percent of the previously detected maximum value, a local research is performed to try to regain the centring. In practice the lamp-holder disk rotates by a small angle in a direction and then in the other one. If the maximum signal is retrieved the waiting step is reached again, otherwise all the procedure is repeated from the beginning. In order to accelerate times for centring again the target, the program implements a kind of "memory of the displacement". I.e. normally the system tries first clockwise and then counter-clockwise, but the priority is decided by experience, since the micro-controller remembers the previous displacement of the target.

The micro-processor card is feed by the same voltage of the lamps (12V) and is provided with filters to suppress noises in the line. The motor is controlled by a power transistor (solid state device in order to prevent sparks) whereas the polarity inversion relay is activated always some tenth of second before starting the motor in order to prevent the formation of voltaic arches and the consequent wear of contacts, as well as for the compatibility of the operating-room, the compatibility being in any case assured as such a relay is sealed.

## Claims

1. A lighting device provided with automatic aiming system, comprising a fixed supporting element (1) and a movable lamp-holder (3) holding light sources (4) that are fixed thereto, driving means adapted to move the movable lamp-holder (3) with respect to the fixed supporting element (1), a sensor and a micro-controller (16) which receives data from the sensor (15) and uses it to control the operation of said driving means in order to modify the position of the movable lamp-holder (3) and then the orientation of light beams thereof in the space, said sensor being an optical sensor (15) adapted to detect a reflectance factor of the light from an illuminated field for each position of the lamp-holder (3), the lighting device also including an optical encoder (13) able to provide the position of the movable lamp-holder (3) in every moment, the micro-controller (16) receiving from the optical sensor (15) data relevant to such a reflectance factor, and from the optical encoder (13) data relevant to the position of the movable lamp-holder (3) to control the operation of driving means for the constant aiming of a zone of the illuminated field which has a maximum reflectance factor, **characterised in that** said driving means comprises a motor (6) having a shaft to which is keyed a driving gear wheel (7), which is meshed with a first driven gear wheel (8), a second driven gear wheel (9) being mounted so to rotate at the same speed and in the same direction of the driving gear wheel (7).

2. The device according to claim 1, **characterised in that** said driving means of the movable lamp-holder (3) comprises a rod (11), which is pivoted at one end thereof to the lamp-holder (3) and, at the other end thereof is pivoted eccentrically to said first driven gear wheel (8) and is constrained to rotate, in a position between its ends, on a lower plate (12) of the frame (2) which is connected to said fixed supporting element (1).

3. The device according to claim 2, **characterised in that** said driving means are mounted to an upper plate (10) of said frame (2).

4. The device according to claim 3, **characterised in that** said frame (2) is attached to said fixed supporting element (1) with the interposition of vibration-damping pads (14) applied to said upper plate (10).

## Patentansprüche

1. Beleuchtungsvorrichtung mit einem automatischen Zielsystem, einschließlich eines fixen Stützelements (1) und eines beweglichen Lampenhalters (3) der Lichtquellen (4) trägt, die darauf befestigt sind, Antriebsmittel um den beweglichen Lampenhalter (3) gegenüber dem fixen Stützelement (1) zu bewegen, einen Sensor und einen MikroKontroller (16) welcher vom Sensor (15) Daten erhält und sie benutzt um den Betrieb der genannten Antriebsmittel zu befehlen um die Lage des beweglichen Lampenhalters (3) und somit die Orientierung seiner Leuchtstrahlen im Raum zu ändern, mit der Eigenschaft dass der genannte Sensor ein optischer Sensor (15) ist, der die Fähigkeit hat, einen Reflexkoeffizient des Lichts vom beleuchteten Feld in jeder Position des Lampenhalters (3) zu bestimmen, die Beleuchtungsvorrichtung schließt auch einen optischen Encoder (13) ein, der in der Lage ist, in jedem Moment die Position des beweglichen Lampenhalters (3) zu geben, der Mikrokontroller (16), der vom optischen Sensor (15) Daten bezüglich diesem Reflektionskoeffizient und vom optischen Encoder (13) Daten bezüglich der Position des beweglichen Lampenhalters (3) erhält um den Betrieb der Antriebsmittel zur konstanten Richtung eines Teils des Beleuchtungsfeldes zu befehlen um einen höchsten Reflektionskoeffizient vorzuweisen, mit der Eigenschaft, dass die genannten Antriebsmittel einen Motor (6) einbeziehen auf dessen Antriebswelle ein Antriebszahnrad (7) im Eingriff mit einem ersten getriebenen Zahnrad (8), einem zweiten getriebenen Zahnrad (9) befestigt und so verzahnt ist, dass es mit derselben Geschwindigkeit und in die selbe Richtung des Antriebszahnrads (7) dreht.

2. Die Vorrichtung gemäß Patentanspruch 1, charakterisiert sich **dadurch**, dass die genannten Antriebsmittel des beweglichen Lampenhalters (3) einen Schaft (11) mit einschließen, welcher an einem Ende am Lampenhalter (3) und, an seinem anderen Ende exzentrisch auf das genannte erste getriebene Zahnrad (8) verzapft ist und gezwungen ist, in einer Stellung zwischen dessen genannten Enden, auf einer unteren Platte (12) eines Rahmens (2) verbunden mit dem genannten fixen Stützelement (1) zu drehen.

3. Die Vorrichtung gemäß Patentanspruch 2, charakterisiert sich **dadurch**, dass die genannten Antriebsmittel auf einer oberen Platte (10) des genannten Rahmens (2) montiert sind.

4. Die Vorrichtung gemäß Patentanspruch 3, charakterisiert sich **dadurch**, dass der genannte Rahmen (2) am genannten fixen Stützelement (1) mit der Zwischenschiebung von vibrations-dämpfenden Kissen (14), die an der genannten oberen Platte (10) angebracht sind, befestigt ist.

## Revendications

1. Dispositif d'éclairage doté d'un système de pointage automatique, comprenant un élément de support fixe (1) et une douille mobile (3) supportant les sources lumineuses (4) qui y sont fixées, des moyens moteurs adaptés pour déplacer la douille mobile (3) par rapport à l'élément de support fixe (1), un capteur et un microcontrôleur (16), qui reçoit les données du capteur (15) et les utilise pour commander l'actionnement desdits moyens moteurs afin de modifier la position de la douille mobile (3), puis l'orientation de ses faisceaux lumineux dans l'espace, ce capteur étant un capteur optique (15) adapté à la détection du facteur de réflectance de la lumière d'un champ éclairé pour chaque position de la douille (3), le dispositif d'éclairage comprenant également un codeur optique (13) à même de fournir la position de la douille mobile (3) à tout moment, le microcontrôleur (16) recevant du capteur optique (15) des données importantes pour ce facteur de réflectance, et du codeur optique (13) des données importantes pour la position de la douille mobile (3) afin de commander l'actionnement des moyens moteurs pour le pointage constant d'une zone du champ éclairé qui a un facteur de réflectance maximum, **caractérisé en ce que** lesdits moyens moteurs comprennent un moteur (6) ayant un arbre sur lequel est assemblée une roue d'engrenage motrice (7), emboîtée à une première roue d'engrenage menée (8), une seconde roue d'engrenage menée (9) étant montée de sorte qu'elle tourne à la même vitesse et dans le même sens que la roue d'engrenage motrice (7).

2. Le dispositif selon la revendication n°1, **caractérisé en ce que** lesdits moyens moteurs de la douille mobile (3) comprennent une tige (11), qui pivote à une extrémité sur la douille (3) et à l'autre extrémité pivote excentriquement sur ladite première roue d'engrenage menée (8) et est contrainte de pivoter dans une position entre ses extrémités, sur une plaque inférieure (12) d'un cadre (2) qui est connecté à l'élément de support fixe (1).

3. Le dispositif selon la revendication 2, **caractérisé en ce que** lesdits moyens moteurs sont montés sur une plaque supérieure (10) dudit cadre (2).

4. Le dispositif selon la revendication 3, **caractérisé en ce que** ledit cadre (2) est attaché au dit élément de support fixe (1) par l'interposition de plots anti-vibratiles (14) appliqués sur ladite plaque supérieure (10).
